# EUROPEAN PATENT SPECIFICATION

(11) **EP 4 186 550 B1**
(45) Date of publication and mention of the grant of the patent: **23.09.2026**
(21) Application number: 21847288.4
(22) Date of filing: 19.07.2021
(51) Int. Cl.: A61M 16/12, A61M 16/16

(54) **MIXED GAS GENERATING SYSTEM**
SYSTEM ZUR ERZEUGUNG VON MISCHGAS
SYSTÈME DE GÉNÉRATION DE GAZ MIXTE

(30) Priority: 23.07.2020 CN 202021468484 U; 23.07.2020 CN 202021464903 U
(43) Date of publication of application: 31.05.2023
(73) Proprietor: Lin, Hsin-Yung, Taoyuan, Taiwan 33341 (TW)
(72) Inventor: Lin, Hsin-Yung, Taoyuan, Taiwan 33341 (TW)
(74) Representative: Wang, Bo
(86) International application number: PCT/CN2021/107050
(87) International publication number: WO 2022/017311

(56) References cited:
- CN-A- 102 068 743
- CN-A- 107 773 828
- CN-A- 107 773 828
- CN-A- 107 810 027
- CN-A- 108 220 994
- CN-A- 109 568 758
- CN-B- 108 531 930
- CN-U- 208 328 128
- KR-B1- 100 803 966
- US-A1- 2018 228 995

## Description

### BACKGROUND OF THE INVENTION

### 1. Field of the invention

The present invention relates to a mixed gas generating device combined with oxygen gas generator, and more particularly, relates to a mixed gas generating device collocating with an oxygen gas generator or connected to a breathing tube to generate a mixed gas of hydrogen gas and oxygen gas.

### 2. Description of the prior art

People are always pay much attention to human life, so many medical technologies are developed to fight disease and keep people alive. Most of the past medical treatment methods are passive; namely, when a disease occurs, the medical treatment, such as surgery, medication and chemotherapy, radiation or recovery from chronic illness, rehabilitation and correction are given. However, in recent years, many medical experts have increasingly concentrated on preventive medical methods, such as healthy food research, genetic disease screening and disease prevention, which have taken the initiative to prevent potential future diseases. In addition, in order to extend human life, numerous anti-aging and anti-oxidant technologies have gradually developed and widely adopted by the public, including skin care products and foods/anti-oxidant drugs.

Researches show that the body's unstable oxygen gas (O+), also known as free radicals (harmful free radicals), caused by various reasons (such as disease, diet, environment or living habits), can mix with inhaled hydrogen gas to form some of the water and be excreted by the body. Namely, by indirectly reducing the number of free radicals in the human body, the reduction of acidic physique is achieved to become healthy alkaline physique, which can resist oxidation and anti-aging, as well as achieve the effects of eliminating chronic diseases and beauty care. Moreover, by increasing the amount of hydrogen gas inhaled, the increase of the time spent on inhaling hydrogen gas (for example, inhaling hydrogen gas during sleeping time) may also efficiently improve the effect of hydrogen gas inhalation.

In addition to the health care mentioned above, hydrogen gas is also used to provide inhalation for patients with lung diseases to relieve symptoms of lung damage. Gas generators comprising an electrolytic cell for producing a hydrogen-oxygen mixed gas are disclosed in CN108531930A CN108531930A and CN107773828A. In the classical hydrogen gas generating device, the concentration of hydrogen gas in the gas produced by the electrolysis of the hydrogen gas generating device is relatively high. Therefore, the hydrogen gas generating device usually uses the method of adding air to dilute the hydrogen gas concentration before producing it for human inhalation. However, when the users are patients with lung disease, their lung functions are low and the patients need to inhale a higher concentration of oxygen gas. Although the previous hydrogen gas generating device can dilute the hydrogen gas concentration with air, it still cannot increase the oxygen gas concentration in the output gas.

### SUMMARY OF THE INVENTION

The present invention is defined in independent claims 1 and 8. Preferred embodiments are matter of the dependent claims.

The embodiments disclosed herein are not to be seen as an extension of the scope of the invention beyond what is defined by the appended claims. The present disclosure provides a mixing generating device to solve the problems of the prior art. In one embodiment, the mixed gas generating device comprises a hydrogen gas generating device and an oxygen gas generator. The hydrogen gas generating device further comprises an electrolytic cell and an integrated water tank module. The electrolytic cell is configured to generate a hydrogen gas and a second oxygen gas when electrolyzing water. The integrated water tank module comprises a hydrogen gas port, an oxygen gas port and an water port coupled to the electrolytic cell to receive the hydrogen gas and the second oxygen gas from the electrolytic cell and supply water to the electrolytic cell. Wherein, the hydrogen gas and the first oxygen gas are mixed to each other to form a mixed gas.

Wherein, the oxygen gas generator is configured outside of the hydrogen gas generating device, the oxygen gas generator comprises an oxygen gas conduit coupled to the hydrogen gas generating device to input the first oxygen gas into the hydrogen gas generating device, so as to mix the hydrogen gas and the first oxygen gas to form the mixed gas.

Wherein, the oxygen gas generator comprises a molecular sieve filtering unit configured to filter an air to generate the first oxygen gas.

Wherein, the mixed gas generating device further comprises a gas mixing tube coupled to the hydrogen gas generating device and the oxygen gas generator to receive the hydrogen gas and the first oxygen gas, so as to mix the hydrogen gas and the first oxygen gas to form the mixed gas.

Wherein, the gas mixing tube is integrated in the integrated water tank module of the hydrogen gas generating device.

Wherein, the gas mixing tube and the oxygen gas generator are configured outside of the hydrogen gas generating device.

Wherein, the mixed gas generating device combined with oxygen gas generator further comprises a backfire preventer disposed between the oxygen gas generator and the gas mixing tube.

Wherein, the hydrogen gas generating device further comprises an atomization/volatile gas mixing tank and a flame arrester. The atomization/volatile gas mixing tank is coupled to the integrated water tank module to receive the hydrogen gas from the integrated water tank module. The atomization/volatile gas mixing tank selectively generates an atomized gas to be mixed with the hydrogen gas, wherein the atomized gas is one or a combination selected from a group consisting of a water vapor, anatomized drops, and an essential oils. The flame arrester is coupled to an entrance of the atomization/volatile gas mixing tank.

Wherein, the electrolytic cell further comprises a cathode chamber, a hydrogen gas output duct, an anode chamber, an oxygen gas output duct, a water input duct and an ion membrane. The cathode chamber is located on a first side of the electrolytic cell. The anode chamber is located on a second side of the electrolytic cell. The ion membrane is disposed between the anode chamber and the cathode chamber. The oxygen gas output duct is coupled to the oxygen gas port. The hydrogen gas output duct is coupled to the hydrogen gas port. The water input duct is coupled to the water port. The anode chamber generates the second oxygen gas and the cathode chamber generates the hydrogen gas when the electrolytic cell electrolyzes water. The oxygen gas output duct is coupled with the anode chamber and penetrates the second side to output the second oxygen gas at the second side, and the hydrogen gas output duct is coupled with the cathode chamber and extends toward the second side and penetrates the second side to output the hydrogen gas at the second side, so as to cause the hydrogen gas and the second oxygen gas to be outputted at the same side of the electrolytic cell.

Another scope of the present disclosure is to provide a mixed gas generating system.

In one embodiment, the mixed gas generating system comprises a hydrogen gas generating device and an oxygen generator. The hydrogen gas generating device further comprises an electrolytic cell, an integrated flow channel module, a condensing filter, a humidification cup and a hydrogen water cup. The electrolytic cell is configured to generate a hydrogen gas when electrolyzing water. The integrated flow channel device is coupled to the electrolytic cell. The condensing filter is engaged with the integrated flow channel device and configured to filter the hydrogen gas generated by the electrolytic cell. The humidification cup is engaged with the integrated flow channel device and configured to humidify the hydrogen gas. The hydrogen water cup is engaged with the integrated flow channel device and configured to accommodate water and selectively receive the hydrogen gas. The hydrogen gas flows into the hydrogen water cup and is mixed with the water contained in the hydrogen water cup to form a hydrogen water. The oxygen gas generator is configured to generate a first oxygen gas. Wherein, the hydrogen gas and the first oxygen gas are mixed to each other to form a mixed gas.

Wherein, the oxygen gas generator is configured outside of the hydrogen gas generating device. The oxygen gas generator comprises an oxygen gas conduit coupled to the hydrogen gas generating device to input the first oxygen gas into the hydrogen gas generating device, so as to mix the hydrogen gas and the first oxygen gas to form the mixed gas.

Wherein, the oxygen gas generator comprises a molecular sieve filtering unit configured to filter an air to generate the first oxygen gas.

Wherein, the mixed gas generating system further comprises a gas mixing tube coupled to the hydrogen gas generating device and the oxygen gas generator to receive the hydrogen gas and the first oxygen gas, so as to mix the hydrogen gas and the first oxygen gas to form the mixed gas.

Wherein, the gas mixing tube is integrated in the integrated water tank module of the hydrogen gas generating device.

Wherein, the gas mixing tube and the oxygen gas generator are configured outside of the hydrogen gas generating device.

Wherein, the mixed gas generating device further comprises a backfire preventer configured between the oxygen gas generator and the gas mixing tube.

Wherein, the hydrogen gas generating device further comprises an atomization/volatile gas mixing tank and a flame arrester. The atomization/volatile gas mixing tank is coupled to the integrated water tank module to receive the hydrogen gas from the integrated water tank module. The atomization/volatile gas mixing tank selectively generates an atomized gas to be mixed with the hydrogen gas, wherein the atomized gas is one or a combination selected from a group consisting of a water vapor, anatomized drops, and an essential oils. The flame arrester is coupled to an entrance of the atomization/volatile gas mixing tank.

Another scope of the present disclosure is to provide a mixed gas generating system.

In one embodiment, the mixed gas generating system comprises a hydrogen gas generating device and a breathing tube. The hydrogen gas generating device further comprises an electrolytic cell and an integrated water tank module. The electrolytic cell is configured to generate a hydrogen gas and a second oxygen gas when electrolyzing water. The integrated water tank module comprises a hydrogen gas port, an oxygen gas port and a water port coupled to the electrolytic cell. The integrated water tank module is configured to receive the hydrogen gas and the second oxygen gas from the electrolytic cell and supply water to the electrolytic cell. The breathing tube is coupled to the oxygen gas generator and configured to receive a first oxygen gas generated by an oxygen generating device configured outside of the hydrogen generating device. Wherein, the hydrogen gas and the first oxygen gas are mixed to each other to form a mixed gas.

Wherein, the mixed gas generating system further comprises a gas mixing tube coupled to the breathing tube to mix the hydrogen gas and the first oxygen gas to form the mixed gas.

Wherein, the gas mixing tube is integrated in the integrated water tank module of the hydrogen gas generating device.

Wherein, the gas mixing tube is configured outside of the hydrogen gas generating device.

Wherein, the mixed gas generating system further comprises a backfire preventer configured in the breathing tube.

Wherein, the hydrogen gas generating device further comprises an atomization/volatile gas mixing tank and a flame arrester. The atomization/volatile gas mixing tank is coupled to the integrated water tank module to receive the hydrogen gas from the integrated water tank module. The atomization/volatile gas mixing tank selectively generates an atomized gas to be mixed with the hydrogen gas, wherein the atomized gas is one or a combination selected from a group consisting of a water vapor, anatomized drops, and an essential oils. The flame arrester is coupled to an entrance of the atomization/volatile gas mixing tank.

Wherein, the electrolytic cell further comprises a cathode chamber, a hydrogen gas output duct, an anode chamber, an oxygen gas output duct, a water input duct and an ion membrane. The cathode chamber is located on a first side of the electrolytic cell. The anode chamber is located on a second side of the electrolytic cell. The ion membrane is disposed between the anode chamber and the cathode chamber. The oxygen gas output duct is coupled to the oxygen gas port. The hydrogen gas output duct is coupled to the hydrogen gas port. The water input duct is coupled to the water port. The anode chamber generates the second oxygen gas and the cathode chamber generates the hydrogen gas when the electrolytic cell electrolyzes water. The oxygen gas output duct is coupled with the anode chamber and penetrates the second side to output the second oxygen gas at the second side, and the hydrogen gas output duct is coupled with the cathode chamber and extends toward the second side and penetrates the second side to output the hydrogen gas at the second side, so as to cause the hydrogen gas and the second oxygen gas to be outputted at the same side of the electrolytic cell.

Another scope of the present disclosure is to provide a mixed gas generating system.

In other embodiment, the mixed gas generating system comprises a hydrogen gas generation device and a breathing tube. The hydrogen gas generated device further comprises an electrolytic cell, an integrated flow channel device, a condensing filter, a humidification cup and a hydrogen water cup. The electrolytic cell is configured to generate a hydrogen gas when electrolyzing water. The integrated flow channel device is coupled to the electrolytic cell. The condensing filter is engaged with the integrated flow channel device. The condensing filter is configured to filter the hydrogen gas generated by the electrolytic cell. The humidification cup is engaged with the integrated flow channel device. The humidification cup is configured to humidify the hydrogen gas. The hydrogen water cup is engaged with the integrated flow channel device. The hydrogen water cup is configured to accommodate water and selectively receive the hydrogen gas. The hydrogen gas being mixed with the water contained in the hydrogen water cup to form a hydrogen water when the hydrogen gas flows into the hydrogen water cup. The breathing tube is coupled to the oxygen gas generator and configured to receive a first oxygen gas generated by an oxygen generating device which is configured outside of the hydrogen generating device. Wherein, the hydrogen gas and the first oxygen gas are mixed with each other to form a mixed gas.

Wherein, the mixed gas generating system further comprises a gas mixing tube coupled to the hydrogen gas generating device and the oxygen gas generator to receive the hydrogen gas and the first oxygen gas, so as to mix the hydrogen gas and the first oxygen gas to form the mixed gas.

Wherein, the gas mixing tube is integrated in the integrated water tank module of the hydrogen gas generating device.

Wherein, the gas mixing tube is configured outside of the hydrogen gas generating device.

Wherein, the mixed gas generating system further comprises a backfire preventer configured between the oxygen gas generator and the gas mixing tube.

Wherein, the hydrogen gas generating device further comprises an atomization/volatile gas mixing tank and a flame arrester. The atomization/volatile gas mixing tank is coupled to the integrated water tank module to receive the hydrogen gas from the integrated water tank module. The atomization/volatile gas mixing tank selectively generates an atomized gas to be mixed with the hydrogen gas, wherein the atomized gas is one or a combination selected from a group consisting of a water vapor, anatomized drops, and an essential oils. The flame arrester is coupled to an entrance of the atomization/volatile gas mixing tank.

In summary, the mixing generating device with oxygen gas generator of present disclosure is capable of generating oxygen gas by the oxygen gas generator and mixing the oxygen gas with the hydrogen gas produced by the electrolytic cell to dilute the hydrogen. The oxygen gas is mixed with the hydrogen gas to generate a mixed gas with a certain concentration of hydrogen gas for the patients with lung diseases to inhale, thus alleviating symptoms and reducing the burden on the lungs. In addition, the oxygen gas inputted by the oxygen gas generator makes the mixed gas having a higher oxygen concentration that is suitable for the patients with damaged lungs who requires high oxygen concentrations. Furthermore, the mixed gas produced by the device of the present disclosure may be mixed with the atomization gas with healing effects to from a healthy gas for human inhalation. Moreover, the device of the present disclosure can prevent the backflow of gas by backfire device, thereby improving safety. Therefore, the mixed gas generating device combined with oxygen gas generator of the present disclosure can provide gas with multiple effects at the same time, improve the symptoms of patients with lung diseases, and also be used for general healthcare.

### BRIEF DESCRIPTION OF THE APPENDED DRAWINGS

FIG 1 is a functional block diagram illustrating a mixing generating device with oxygen gas generator according to an embodiment of the present disclosure.
FIG 2 is a partial schematic diagram illustrating a mixing generating device with oxygen gas generator according to another embodiment of the present disclosure.
FIG 3 is a cross-section diagram illustrating the mixing generating device with oxygen gas generator in FIG 2.
FIG 4 is a sectional diagram illustrating the electrolytic cell in FIG 2.
FIG 5 is a schematic diagram illustrating a mixed gas generating device according to an embodiment of the present disclosure.
FIG 6 is a schematic diagram illustrating a mixed gas generating device according to another embodiment of the present disclosure.
FIG 7 is an explosion diagram illustrating a hydrogen gas generating device according to an embodiment of the present disclosure.
FIG 8 is a schematic diagram illustrating the hydrogen gas generating device according in FIG 7 from another view angle.
FIG 9 is a schematic diagram illustrating a mixed gas generating device according to another embodiment of the present disclosure.
FIG 10 is a functional block diagram illustrating a mixed gas generating device connected with a breathing tube and an oxygen gas generator according to an embodiment of the present disclosure.
FIG 11a is a schematic diagram illustrating a mixed gas generating system according to an embodiment of the present disclosure.
FIG 11b is a schematic diagram illustrating a mixed gas generating system according to another embodiment of the present disclosure.
FIG 12 is a partial schematic diagram illustrating the mixed gas generating device connected with the breathing tube in FIG 11a.
FIG 13 is a sectional diagram illustrating the mixed gas generating device in FIG 11a.
FIG 14 is a schematic diagram illustrating a mixed gas generating system according to another embodiment of the present disclosure.
FIG 15 is a schematic diagram illustrating a mixed gas generating system according to another embodiment of the present disclosure.

### DETAILED DESCRIPTION OF THE DISCLOSURE

For the sake of the advantages, features of the present disclosure can be understood more easily and clearly, the detailed descriptions and discussions will be made later by way of the embodiments and with reference of the diagrams. It is worth noting that these embodiments are merely representative embodiments of the present disclosure, wherein the specific methods, devices, conditions, materials and the like are not limited to the embodiments of the present disclosure or corresponding embodiments. Moreover, the devices in the figures are only used to express their corresponding positions and are not drawing according to their actual proportion.

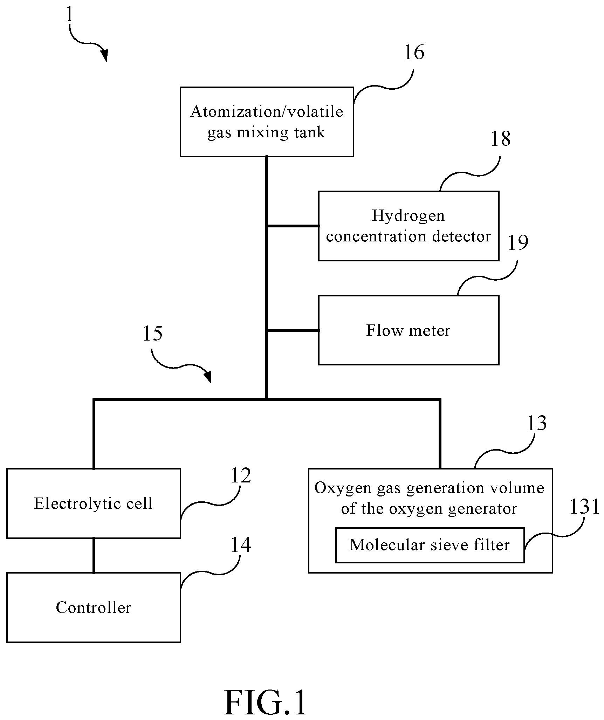

Please refer to FIG 1. FIG.1 illustrates a functional block diagram of the mixing generating device with oxygen gas generator 1 according to an embodiment of the present disclosure. The mixed gas generating device combined with oxygen gas generator 1 comprising an electrolytic cell 12, an oxygen gas generator 13, and a gas mixing tube 15. The electrolytic cell 12 is configured for electrolyzing water to produce a hydrogen gas-containing gas or the hydrogen gas. The gas mixing tube 15 is coupled to the electrolytic cell 12 and the atomization/volatile gas mixing tank 16 for transporting the hydrogen gas-containing gas or hydrogen gas generated by the electrolytic cell 12 to the atomizing gas/volatile gas mixing tank 16. The oxygen gas generator 13 comprises a molecular sieve filter 131 to filter the air and generate the oxygen gas, which is called a first oxygen gas hereafter. Besides, the oxygen gas generator 13 is coupled to the gas mixing tube 15 to dilute the hydrogen gas by inputting the first oxygen gas into the gas mixing tube 15 to form a mixed gas.

In this embodiment, the mixed gas generating device combined with oxygen gas generator 1 further comprises a hydrogen concentration detector 18 coupled with the gas mixing tube 15 to detect a gas volume concentration in the gas mixing tube 15. In practice, the hydrogen concentration detector 18, the electrolytic cell 12, and the oxygen gas generator 13 can be coupled to the controller 14 of mixed gas generating device combined with oxygen gas generator 1. The controller 14 can respectively adjust the hydrogen gas generation production quantity of the electrolytic cell 12 and the oxygen gas production quantity of the oxygen generator 13, according to the hydrogen concentration detected by the hydrogen concentration detector 18, so as to dilute and adjust the hydrogen concentration of the mixed gas in the gas mixing tube 15 to be less than a predetermined value for human inhalation. In practice, the predetermined value can be 1% to 7.5%, but not limited to, the inhalation of hydrogen concentration depends on user's body.

In this embodiment, the mixed gas generating device combined with oxygen gas generator 1 further comprises the flow meter 19. The flow meter19 coupled to the gas mixing tube 15 to detect the flow rate value of the mixed gas in the gas mixing tube 15. In practice, the flow meter 19 also can be coupled to the controller 14 as mentioned above, the controller 14 can respectively adjust the hydrogen gas generation volume of the electrolytic cell 12 and the oxygen gas generation volume of the oxygen generator 13 according to the flow rate value of the mixed gas detected by the flow meter19. Furthermore, the mixed gas generating device combined with the oxygen gas generator 1 generates the mixed gas at the predetermined flow value for the user inhalation. In practice, the predetermined flow value of the mixed gas can be 1.0 L/min to 6.0 L/min or above 6.0 L/min. In an embodiment, the predetermined flow value of the mixed gas can be between 3.0 L/min to 6.0 L/min.

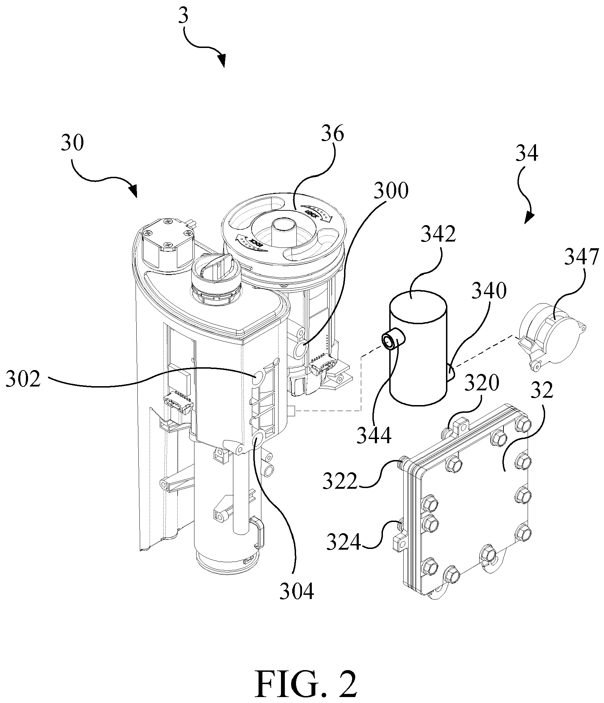

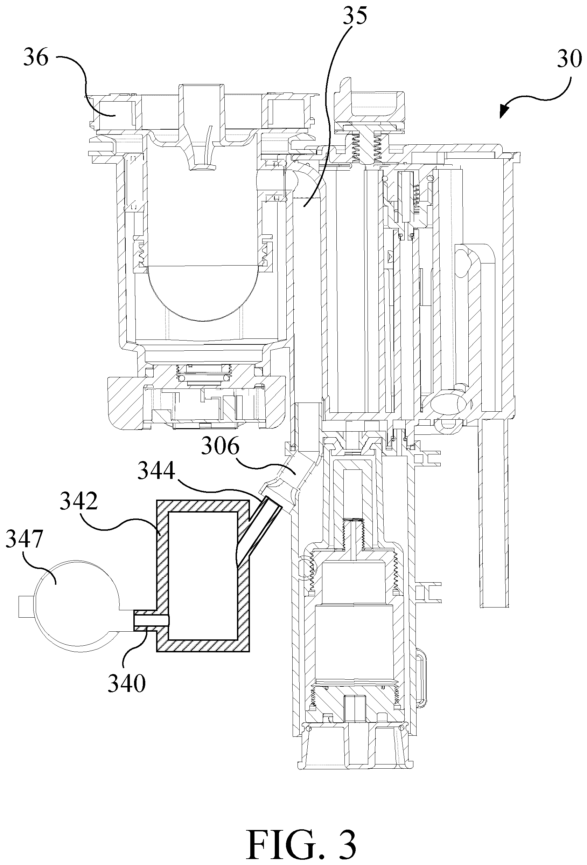

Please refer to FIG2 and FIG3. FIG2 illustrates a partial graph of the mixing generating device with oxygen gas generator according to another embodiment of the present disclosure. FIG.3 illustrates a cross-section of a mixing generating device with oxygen gas generator in FIG2. Please notice that the FIG2 shows a partial of the mixing generating device with oxygen gas generator, and other parts are omitted for brevity, such as casing or other units within the casing. As shown in FIG 2, the mixing generating device with oxygen gas generator 3 comprises the integrated water tank module30, the electrolytic cell 32, the oxygen gas generator 34, and the atomization/volatile gas mixing tank 36. Furthermore, the integrated water tank module 30 comprises the sink, the gas mixing tube 35, the hydrogen port 300, the oxygen port 302, and the water port 304. Wherein, the hydrogen port 300 is coupled to the gas mixing tube 35 within the integrated water tank module 30, and the oxygen port 302 and water port 304 are coupled to the sink within the integrated water tank module 30. The hydrogen port 300, the oxygen port 302, and the water port 304 of the integrated water tank module 30 can be separately coupled to hydrogen gas output duct 320. The oxygen gas output duct 322 and the water input duct 324 of the electrolytic cell 32 are to respectively receive the hydrogen gas and the oxygen gas generated by the electrolytic cell 32 to supplement the electrolyzed water in the electrolytic cell 32. Furthermore, the second oxygen output of the electrolytic tank 32 may be mixed with little residual electrolyzed water, and the residual electrolyzed water remains in the water tank of the integrated water tank module 30 for recycling.

In this embodiment, the oxygen gas generator 34 further comprises the air conduit 340, the molecular sieve oxygen generator 342, the oxygen gas conduit 344, and the air pump 347. In practice, the molecular sieve filter unit 342 can be formed by a plurality of molecular sieves in the container, such as a molecular sieve filter to filter out oxygen gas in the air. Specifically, the molecular sieves adsorb gases other than oxygen gas into the air, and only allow oxygen gas to pass through. In this embodiment, the air conduit 3 is coupled to the air pump 347 and the molecular sieve oxygen generator 342; the oxygen gas conduit 344 is coupled to the molecular sieve oxygen generator 342 and the gas mixing tube 35 of the integrated sink module 30. The air pump 347 sucks in air from the environment and passes the air through the air conduit 340 to the molecular sieve filter unit 342. The joint position between the supplemental gas conduit 306 and the gas mixing tube 35 has an angle, and the joint position must form an arc lead angle. In practice, the angle may be a sharp angle below 90 degrees, and a better angle range in the design is between 25 and 45 degrees. When the air conduit 340 transmits the air sucked by the air pump 347 to the molecular sieve filter unit 342, the molecular sieve filter unit 342 filters out the first oxygen gas in the air, and outputs the first oxygen gas to the supplemental gas conduit 306 through the oxygen gas conduit 344. The supplemental gas conduit 306 is designed with an angle so that the first oxygen gas in the oxygen gas conduit 344 enters into the gas mixing tube 35 to dilute the hydrogen gas in the gas mixing tube 35. The oxygen gas generator can be installed outside of the hydrogen generating device or inside it.

In this embodiment, the atomization/ volatile gas mixing tank 36 is coupled to the gas mixing tube 35 to receive the mixed gas including the hydrogen gas and oxygen gas. The atomization/ volatile gas mixing tank 36 generates an atomizing gas to be mixed with the mixed gas to form a healthy gas, wherein the atomizing gas is one or a combination selected from a group consisting of water vapor, anatomized drops, and essential oils. In one embodiment, the atomization/volatile gas mixing tank 36 comprises an oscillator. The oscillator atomizes water, anatomized drops and essential oils in the atomization/volatile gas mixing tank 36 through oscillation to generate atomized gas, and to mix the gas with the atomized gas to form a health gas. The atomization/volatile gas mixing tank 36 can be selectively turned on or off according to users' needs to provide healthcare gas mixed with atomized gas or mixed gas (such as the hydrogen gas diluted by the second oxygen gas) for users to inhale.

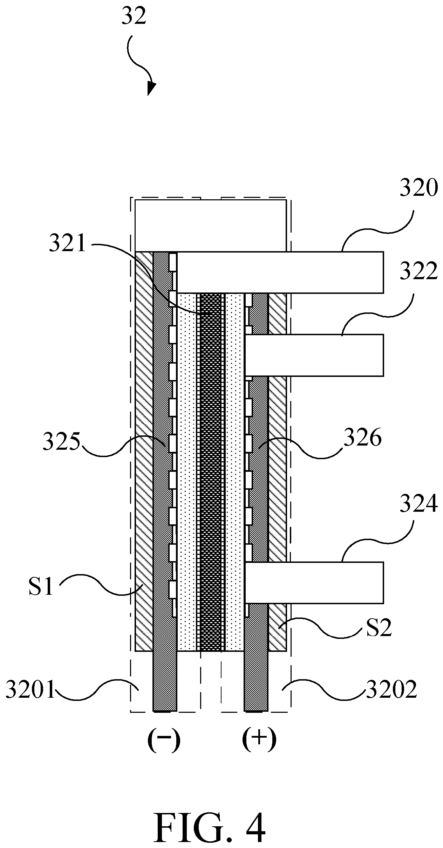

Please refer to FIG2 and FIG4. FIG4 illustrates a cross-section of the electrolytic cell in FIG2. In this embodiment, the electrolytic cell 32 can be an ion membrane electrolytic cell. In practice, it is not limited to an ion membrane electrolytic cell. This present disclosure may also use other types of electrolytic cells.

As shown in FIG 4. The electrolytic cell 32 comprises an ion membrane 321, a cathode chamber 325, an anode chamber 326, a first side S1, a second side S2, a hydrogen gas output duct 320, and an oxygen gas output duct 322. The ion membrane 321 is disposed between the first side S1 and the second side S2; the cathode 325 is disposed between the ion membrane 321 and the first side S1; and the anode 326 is disposed between the ion membrane 321 and the second side S2. Wherein the area of the first side S1 and the cathode 325 located is called the cathode chamber 3201, and the area of the second side S2 and the anode electrode 326 located is called the anode chamber 3202. In order to better express the corresponding positions for the cathode chamber 3201 and the anode chamber 3202, the corresponding positions are shown by a dotted line in the FIG 3. The hydrogen gas output duct 320 extends from between the ion membrane 321 and the first side S1 to the second side S2 and penetrates the second side S2, and the oxygen gas output duct 322 extends between the ion membrane 321 and the second side S2, and penetrates the second side S2. The cathode chamber generates the hydrogen gas and the anode chamber generates the second oxygen gas when the electrolytic cell electrolyzes water. In practice, the second oxygen gas contains oxygen, and may also contain the ozone that is not conducive to human inhalation, so the second oxygen gas and hydrogen gas are outputted separately. In this embodiment, the hydrogen gas and the second oxygen gas generated by the electrolytic cell 32 electrolyzing water are outputted from the second side S2 of electrolytic cell 32 respectively through the hydrogen gas output duct 320 and oxygen gas output duct 322. Furthermore, the electrolytic cell 12 may comprise the water input duct 324 coupled to the anode chamber 3202 and penetrate the second side S2, to receive water to supplement the electrolyzed water lost after electrolysis. Therefore, the water input duct 324, the hydrogen gas output duct 320, and the oxygen gas output duct 322 are disposed on the second side S2 of the electrolytic cell 32.

In this embodiment, the hydrogen gas output duct 320, the oxygen gas output duct 322 and the water input duct 324 are disposed on one side of the anode chamber 3202 of the electrolytic cell 32 (the second side, S2), but it is not limited to this. In one embodiment, the hydrogen gas output duct 320, the oxygen gas output duct 322 and the water input duct 324 can be disposed on one side of the cathode chamber 3201 of the electrolytic cell 32 (the first side S1).

The mixing generating device with oxygen gas generator of the present disclosure can also include a backflow preventer device (The figure is not shown), disposed between the oxygen gas generator and the gas mixing tube, configured to prevent gas in the gas mixing tube from flowing through the oxygen generator. In practice, the backflow preventer device can be a flame arrester. The flame arrester comprises the non-return valve; therefore, the gas can pass through in one direction only. In another one of embodiment, the backflow preventer device is disposed on the gas mixing tube and is close to the oxygen generator, so that the first oxygen gas in the oxygen gas duct can flow to the gas mixing tube through the backflow preventer device, and the gas in the gas mixing tube cannot flow into the oxygen gas duct. Because the gas mixing tube may contain inflammable gas (such as hydrogen), the backflow preventer device can prevent other gas in the gas mixing tube from returning back to the oxygen gas duct, thereby preventing the gas unfortunately ignited from spreading to the oxygen generator and then improving safety. The location of the backflow preventer device is not limited to the place mentioned above. In one embodiment, the backflow preventer device can be disposed on the oxygen gas duct of the oxygen generator and close to the gas mixing tube. In another one of embodiment, the backflow preventer device is disposed between the gas mixing tube and the atomizing gas/volatile gas mixing tank, and is disposed at the entrance of the atomizing gas/volatile gas mixing tank, to prevent the gas of the atomizing gas/volatile gas mixing tank from backflowing to the gas mixing tube. In one embodiment, the flame arrester can contain at least one of a metal mesh filter and a corrugated filter. The metal mesh filter can be the stainless steel or the copper mesh with a diameter of 0.23~0.315mm and composed of multiple layers. The corrugated filter can be stainless steel, copper-nickel alloy, aluminum, and aluminum alloy which can be used to prevent the violent flame of deflagration and withstand the corresponding thermal and mechanical effects. The flame arrestor is used to block off the fire from flowing to the flame arrestor, thereby isolating the two spaces to avoid the fire spreading from one side of the flame arrester to the other side, which can cause the fire to spread through the gas flow path and make an explosion.

In practice, the mixed gas generating device combined with an oxygen generator of the aforementioned embodiments further comprises a control panel coupled to the above-mentioned of the controller, thereby controlling the electrolytic cell and the oxygen generator and adjusting the hydrogen volume concentration and the flow rate of the mixed gas output.

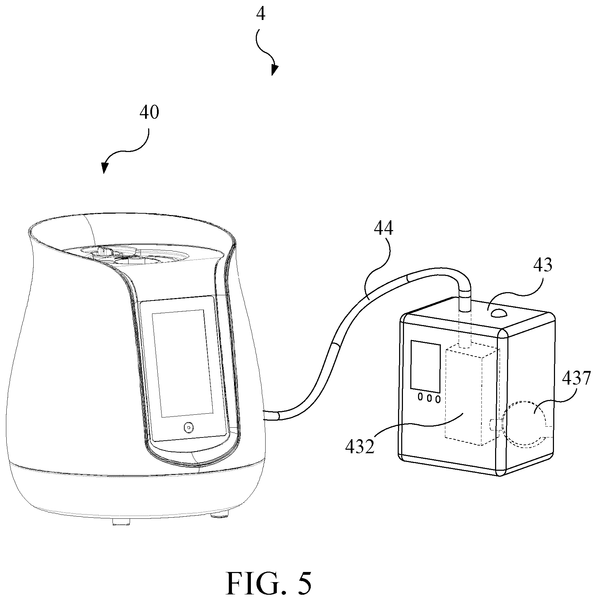

The mixing generating device with the oxygen gas generator of the present disclosure not only can be the aforementioned embodiments, but also can be other forms. Please refer to FIG 5. FIG 5 illustrates a schematic diagram of a mixed gas generating device 4 according to an embodiment of the present disclosure. As shown in FIG 5, the difference between this embodiment and the aforementioned embodiment is that the oxygen generator 43 is coupled to hydrogen generating device 40 from the outside of the hydrogen generating device 40. In this embodiment, the hydrogen generating device 40 comprises the casing, and the casing includes an accommodating space for the electrolytic cell, the integrated sink module, the atomization/ volatile gas mixing tank, and the gas mixing tube mentioned above. The oxygen generator 43 is disposed on the outside of the hydrogen generating device 40, comprising the molecular sieve filtering unit 432, the air pump 437 and the oxygen gas conduit 44 mentioned above (the molecular sieve filtering unit 432 and the air pump 437 are represented by the dashed lines in figure). The oxygen conduit 44 passes through the casing of the hydrogen generating device 40 from the outside of the hydrogen generating device 40 and coupled to the gas mixing tube located inside the casing of the hydrogen generating device 40, and the gas mixing tube is coupled to the atomization/ volatile gas mixing tank. Therefore, the oxygen generator 43, through the molecular sieve filter unit 432, inputs the first oxygen generated by the air pump 437 into the gas mixing tube to dilute the hydrogen gas to form a mixed gas.

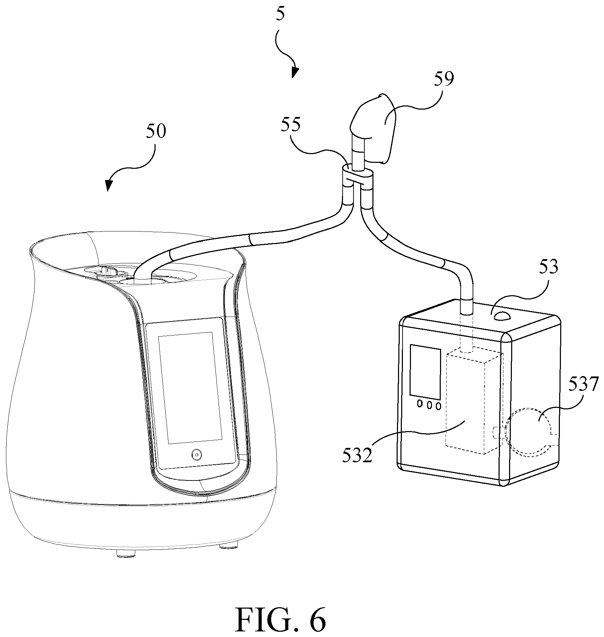

Please refer to FIG 6. FIG 6 illustrates a schematic diagram of a mixed gas generating device according to another embodiment of the present disclosure. As shown in FIG 6, the difference between this embodiment and the aforementioned embodiment is that the gas mixing tube 55 and the oxygen generator 53 are both coupled to the hydrogen generating device 50 from the outside of the hydrogen generating device 50. In this embodiment, the hydrogen generating device 50 comprises a casing, and the casing includes an accommodating space for the electrolytic cell, the integrated sink module, and the atomization/volatile gas mixing tank mentioned above. The atomization/ volatile gas mixing tank comprises a gas port, and the gas mixing tube 55, from the outside of the casing of the hydrogen generating device 50, is coupled to the gas port of the atomization/volatile gas mixing tank, the oxygen generating device 53, and the breathing mask 59. The oxygen generator 53 is disposed on the outside of the hydrogen generating device 50, and can comprise the molecular sieve filtering unit 532, the air pump 537 mentioned above (the figure is represented by a dashed line). The electrolytic cell of the hydrogen generating device 50 generates hydrogen gas or hydrogen-containing gas and outputs the hydrogen gas or hydrogen-containing gas to the gas mixing tube 55 through the atomization/volatile gas mixing tank. The oxygen generator 53, through the molecular sieve filter unit 532, inputs the first oxygen generated by the air pump 537 into the gas mixing tube 55 to form a mixed gas, so that users can inhale the mixed gas through the breathing mask 59.

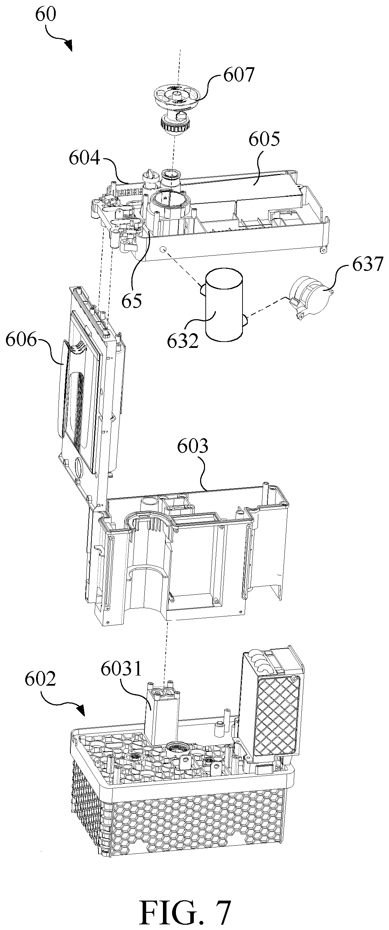

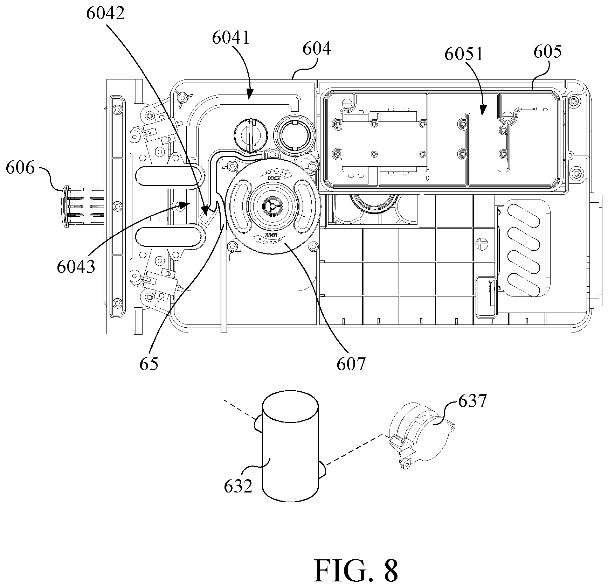

Please refer to FIG 7 and FIG8. FIG 7 illustrates an explode diagram of a hydrogen gas generating device according to embodiment of the present disclosure. FIG8. illustrates a schematic diagram with different visual angles of a hydrogen gas generating device according in FIG7. As shown in FIG 7 and FIG 8., in the embodiment, the hydrogen gas generating device 60 comprises the electrolytic cell (the figure is not shown), the water tank 602, the humidification cup 603, the integrated flow channel 604, the condensing filter devices 605, hydrogen water cup 606 and the atomization/volatile gas mixing tank 607. The electrolytic cell can be an electrolytic cell of electrode type contained in the water tank 602, and receive the electrolyzed water from the water tank 602 for being electrolyzed to generate hydrogen-containing gas. The humidification cup 603 is vertically stacked on the water tank 602; the integrated flow channel 604 is vertically stacked on the humidification cup 603; and the condensing filter devices 605 is contained in the accommodation space of the integrated flow channel 604.

The condensing filter devices 605 is used to filter the hydrogen gas and comprises the condensing flow channel 6051. In practice, the condensing filter devices 605 can be implanted to the integrated flow channel 604 and can be drawn out from the side of the integrated flow channel 604 for replacement without disassembling the entire gas generating device 60. The humidification cup 603 comprises the humidification space (the figure is not shown) and the connected space 6031. The humidification space comprises the supplemental water and can be used to humidify the hydrogen-containing gas. The connected space 6031 is coupled to the water tank 602 and the integrated flow channel 604; therefore, the hydrogen generated by the electrolytic cell disposed in the water tank 602 enters into the condensing flow channel 6051 of the condensing filter device 605. The hydrogen water cup 606 can be used to contain drinking water and input the hydrogen gas into the drinking water to form hydrogen-containing water. The integrated flow channel 604 comprises the enter gas flow channel 6041, the exit gas channel 6042, and the gas connecting flow channel 6043. Wherein the enter gas flow channel 6041 and the exit gas flow channel 6042 can be selectively coupled to the hydrogen water cup 606, and the gas connecting flow channel 6043 can be selectively coupled to the enter gas flow channel 6041 and the exit gas channel 6042. The atomization/volatile gas mixing tank 607 comprises the exit gas channel 6042 to receive the hydrogen gas, and produces the atomizing gas mixing with hydrogen gas to form a health-care gas. In additional, the humidification cup 603, the condensing filter device 605, the atomization/volatile gas mixing tank 607, and the hydrogen water cup 606 can be engaged with or coupled to the integrated flow channel device 604. Furthermore, the hydrogen gas generating device 60 also comprises the aforementioned flame arrestor (the figure is not shown), located at the entrance of the atomizing gas/volatile gas mixing tank 607.

Therefore, the hydrogen gas generated by the electrolytic cell leaves the water surface of the water tank 602 and quickly enters into the connected space 6031 of the humidification cup 603. Then the hydrogen gas flows through the connected space 6031 of the humidification bottle 603, the condensation flow channel 6051 of the condensation filter device 605, and the enter gas flow channel 6041, the exit gas flow channel 6042, and the atomization/volatile gas mixing tank 607 of the integrated flow channel 604. Wherein, the hydrogen gas can selectively flow through the hydrogen water cup 606. However, it should be understood that the above-mentioned flow direction of the hydrogen-containing gas is one of the embodiments of the hydrogen gas generator E of the present disclosure, those skilled in the art can adjust the order of the components according to their needs, and it is not limited to this.

In this embodiment, the hydrogen gas generating device 60 comprises the molecular sieve filter unit 632 and the air pump 673, and the molecular sieve filter unit 632 is used to filter the air sucked by the air pump 637 and generate oxygen. The gas mixing tube 65 can be connected to the exit gas channel 6042 of the integrated flow channel device 604 and receive the hydrogen output from the exit gas channel 6042, and the gas mixing tube 65 can be connected to the molecular sieve filter unit 632 and the air pump 637. The functions of the molecular sieve filter unit 632 and the air pump 637 of this embodiment are substantially the same as the functions of the molecular sieve filter unit and the air pump of the aforementioned embodiment, so that it is not repeated herein. The gas mixing tube 65 can receive the hydrogen gas output from the exit gas channel 6042, and the oxygen gas generates by the molecular sieve filter unit 632 to form a mixed gas. The atomizing/volatile gas mixing tank 607 of the hydrogen gas generating device 60 can be connected to the gas mixing tube 65 and receive the mixed gas in the gas mixing tube 65, and the atomizing/volatile gas mixing tank 607 can generate the atomizing gas and the mixed gas to form the health care gas. The oxygen generator 63 can be disposed outside the casing 601 of the hydrogen gas generating device 60, or disposed inside the casing 601.

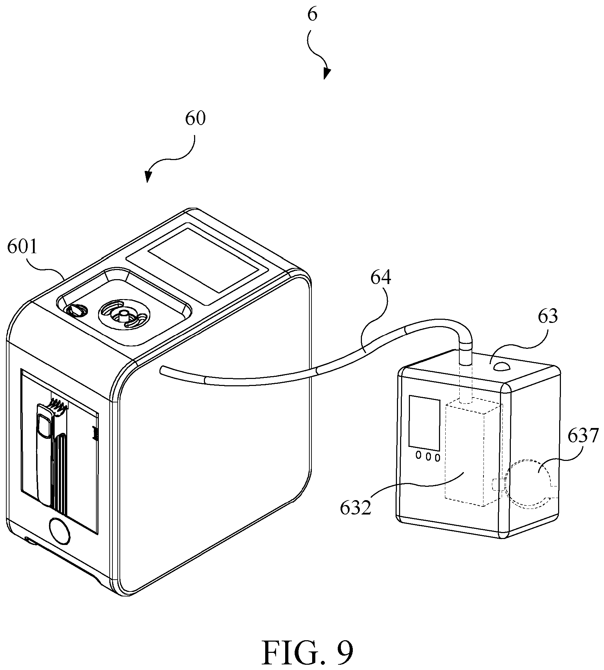

Please refer to FIG 9. FIG 9 illustrates a schematic diagram of a mixed gas generating device according to another embodiment of the present disclosure. In this embodiment, the hydrogen gas generating device 60 comprises the casing 601; the gas mixing tube 65 is disposed in the casing 601 of the hydrogen gas generating device 60, and the oxygen generator 63 is disposed outside the hydrogen gas generating device 60. The oxygen gas generating device 63 comprises the aforementioned molecular sieve filter unit 632, the air pump 637, and an oxygen conduit 64 (the molecular sieve filter unit 632 and the air pump 637 in the figure are represented by dashed lines). The oxygen conduit 64 of the oxygen generator 63 passes through the casing 601 of the hydrogen generator 60 from the outside of hydrogen generator 60, and is coupled with the gas mixing tube 65 in the casing 601 of the hydrogen generator 60. Therefore, the oxygen generator 63, through the molecular sieve filter unit 632, inputs the first oxygen generated by the air pump 437 into the gas mixing tube 65 to dilute the hydrogen gas to form a mixed gas. In another embodiment, the gas mixing tube and the oxygen generator both are disposed outside of the hydrogen gas generating device. The atomization/volatile gas mixing tank is coupled to the exit gas channel of the integrated flow channel device, and the gas mixing tube is coupled to the atomizing/volatile gas mixing tank.

In the above-mentioned embodiments, the mixed gas generating device or system comprise the oxygen generator such as molecular sieve filter to mix the hydrogen gas generated by the electrolytic cell with the oxygen gas generated by the oxygen generator to form the mixed gas for users to inhale. However, the mixed gas generating device can also be connected to an oxygen generator or an oxygen supply which is configured outside of the mixed gas generating device, such as the oxygen supply for the patient in hospital, so as to extend the use of the mixed gas generating device or system in the present disclosure.

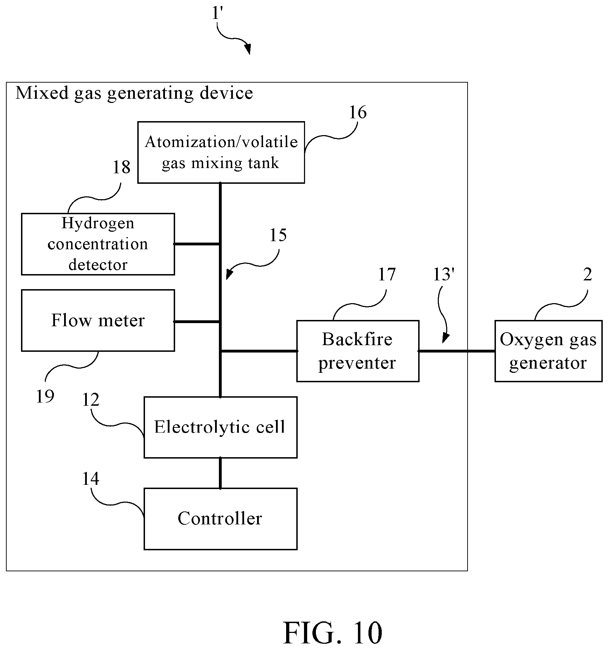

Please refer to FIG 10. FIG 10 is a functional block diagram illustrating a mixed gas generating device connected with breathing tube and an oxygen gas generator according to an embodiment of the present disclosure. As shown in FIG 10, the mixed gas generating device connected with the breathing tube 1' comprises an electrolytic cell 12, a gas mixing tube 15 and the breathing tube 13'. The electrolytic cell 12 is configured for electrolyzing water to generate a gas comprising hydrogen or a hydrogen gas. The gas mixing tube 15 is coupled to the electrolytic cell 12 and the atomized/volatile gas mixing tank 16 for transporting the gas comprising hydrogen or the hydrogen gas generated by the electrolytic cell 12 to the atomized/volatile gas mixing tank 16. The breathing tube 13' is coupled to the gas mixing tube 15 and the oxygen gas generator 2, and is configured to receive the oxygen gas provided by the oxygen gas generator 2 and then inputs the oxygen to the gas mixing tube 15, so as to dilute the hydrogen gas or the gas comprising hydrogen in the gas mixing tube 15 to form the mixed gas. The oxygen gas inputted by the breathing tube 13' is called as the first oxygen hereafter.

In this embodiment, the mixed gas generating device connected with breathing tube 1 further comprises a hydrogen concentration detector 18. The hydrogen concentration detector 18 is coupled to the gas mixing tube 15 to detect a hydrogen volume concentration in the gas mixing tube 15. In practice, the hydrogen concentration detector 18 and the electrolytic cell 12 can be coupled to a controller 14 of the mixed gas generating device connected with breathing tube 1'. The controller 14 can respectively adjust the hydrogen gas generating quantity of the electrolytic cell 12 according to the hydrogen concentration detected by the hydrogen concentration detector 18, so as to dilute and adjust the hydrogen concentration of the mixed gas in the gas mixing tube 15 to be less than a predetermined value for human inhalation. The predetermined value can be 1%, 2%, 3%, 4%, 4.5%, 5%, 5.5%, 6%, 6.5%, 7% or 7.5%. However, the hydrogen concentration of the mixed gas is not limited to the above values but can be determined by the requirements of the user's body.

In this embodiment, the mixed gas generating device connected with breathing tube 1' further comprises a flow meter 19. The flow meter 19 is coupled to the gas mixing tube 15 to detect the flow rate value of the mixed gas in the gas mixing tube 15. In practice, the flow meter 19 also can be coupled to the controller 14. The controller 14 can adjust the quantity of the hydrogen gas generated by the electrolytic cell 12 according to the flow rate value of the mixed gas detected by the flow meter 19, so that the mixed gas generating device connected with breathing tube 1' generates the mixed gas with a predetermined flow value for the user to inhale. In practice, the predetermined flow value of the mixed gas can be 1.0 L/min, 2.0 L/min, 3.0 L/min, 4.0 L/min, 5.0 L/min, 6.0 L/min or above 6.0 L/min. In an embodiment, the predetermined flow value of the mixed gas can be between 3.0 L/min to 6.0 L/min.

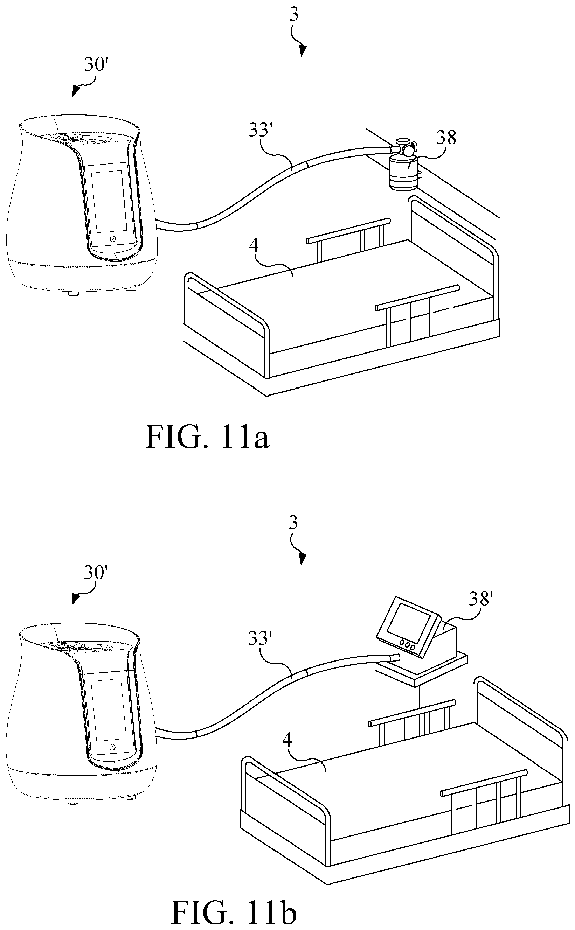

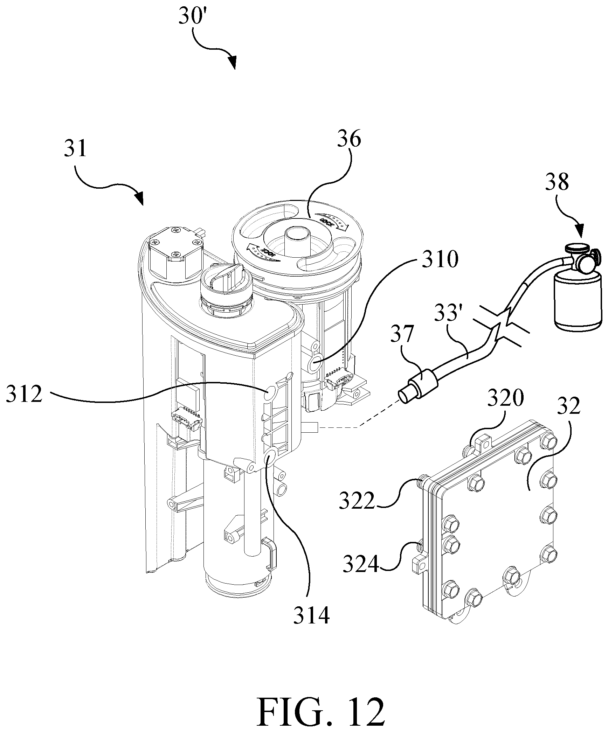

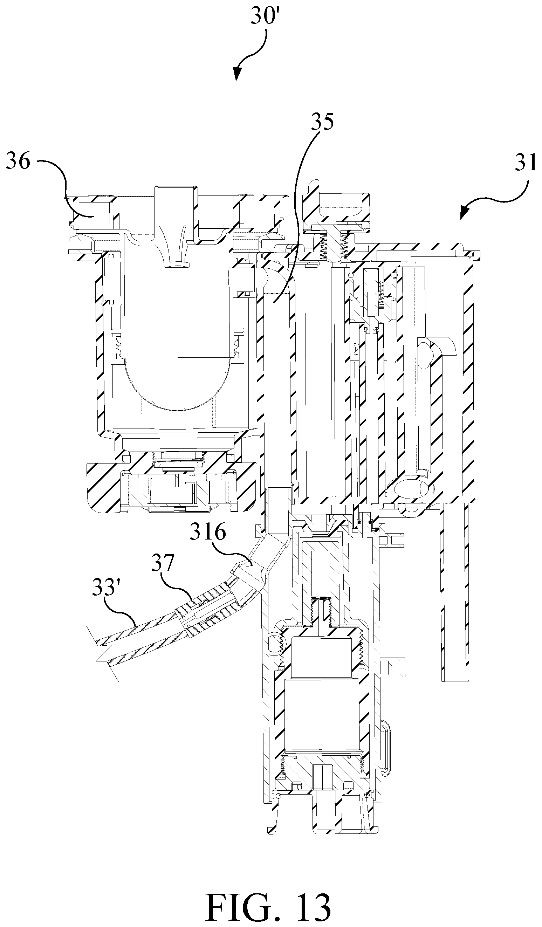

Please refer to FIG 11a, FIG 11b, FIG 12 and FIG 13. FIG 11a is a schematic diagram illustrating a mixed gas generating system according to an embodiment of the present disclosure. FIG. 11b is a schematic diagram illustrating a mixed gas generating system according to another embodiment of the present disclosure. FIG. 12 is a partial schematic diagram illustrating the mixed gas generating device connected with the breathing tube 30' in FIG 11a. FIG 13 is a sectional diagram illustrating the mixed gas generating device 30' in FIG 11a. It should be noted that FIG 12 and FIG 13 show a part of the mixing generating device connected with the breathing tube 30', and the other parts, such as the casing or other units within the casing, are omitted from these figures for sake of brevity. In this embodiment, the mixed gas generating device connected with breathing tube 30' further comprises an integrated water tank module 31, the electrolytic cell 32, the breathing tube 33', and the atomized/volatile gas mixing tank 36. The mixed gas generating device connected with breathing tube 30' is coupled to an oxygen generating device 38. Furthermore, the integrated water tank module 31 comprises a water tank and a gas mixing tube 35. One end of the breathing tube 33' is coupled to the gas mixing tube 35, and a part of breathing tube 33' extends to the outside of the casing of the mixed gas generating device connected with the breathing tube 30' to be coupled to the oxygen generating device 38, so as to input the first oxygen generated by oxygen generating device 38 through the breathing tube 33' into the gas mixing tube 35 of the mixed gas generating device connected with breathing tube 30' to dilute the hydrogen gas to form a mixed gas. In practice, the oxygen generating device 38 can be the equipment for hospital or medical use (as shown in FIG 11a, the oxygen equipment 38 corresponding to the hospital bed 4), the oxygen bottle or the ventilator 38' (as shown in FIG 11b). The breathing tube 33' can be the oxygen tube for hospital or medical use, or the output tube of the ventilator.

As show in FIG 12 and FIG 13. The integrated water tank module 31 further comprises a hydrogen gas port 310, an oxygen gas port 312, and a water port 314. The hydrogen gas port 310 coupled to the gas mixing tube 35 within the integrated water tank module 31 and the oxygen gas port 312, the water port 314 are coupled to the sink within the integrated water tank module 31. The hydrogen port 310, the oxygen port 312, and the water port 314 of the integrated water tank module 31 can be separately coupled to the hydrogen gas output duct 320, the oxygen gas output duct 322 and the water input duct 324, configured to respectively receive the hydrogen gas and the oxygen gas generating by the electrolytic cell 32, and output the water to supplement the electrolyzed water in the electrolytic cell 32. The detailed descriptions of the hydrogen gas output duct 320, the oxygen gas output duct 322 and the water input duct 324 of the electrolytic cell 32 will be made later. Furthermore, the integrated water tank module 31 comprises a supplement gas port 316 coupled to the breathing tube 33' and the gas mixing tube 35. The joint position between the supplement gas port 316 and the gas mixing tube 35 has an angle, and the joint position must form an arc lead angle. In practice, the angle may be a sharp angle below 90 degrees, and a better angle range in the design is between 25 and 45 degrees. The supplemental gas port 316 is designed with an angle so that the first oxygen in the breathing tube 33' enters into the gas mixing tube 35 to dilute the hydrogen in the gas mixing tube 35.

In addition, the gas mixing tube within the integrated water tank module 31 is coupled to the atomized/volatile gas mixing tank 36, so that the hydrogen gas outputted from the electrolytic cell 32 enters into the gas mixing tube within the integrated water tank module 30 and then further goes into the atomized/volatile gas mixing tank 36. In practice, the oxygen gas (called as the second oxygen in the following) generated by the electrolytic cell 32 thought the oxygen gas output duct 322 and the oxygen port 312 is directly outputted to the integrated water tank module 31, and is discharged to the atmosphere. Furthermore, the second oxygen outputted by the electrolytic tank 32 may be mixed with little residual electrolyzed water, and the residual electrolyzed water remains in the water tank of the integrated water tank module 30 for recycling.

Please refer to FIG 10 and FIG 13. The mixed gas generating device connected with the breathing tube 1' of the present disclosure further comprises a backfire preventer 17 disposed between the gas mixing tube 15 and the breathing tube 13'. As shown in FIG 13, in this embodiment, the backfire preventer 37 is disposed in the breathing tube 33' and is configured to prevent gas in the gas mixing tube 15 from flowing to the breathing tube 33'. The backflow preventer 37 can be the flame arrester. The flame arrester comprises the non-return valve; therefore, the gas can pass through in one direction only. In this embodiment, the backflow preventer 37 is disposed on the side of the gas mixing tube 35 and is close to the breathing tube 33'; therefore the first oxygen in the breathing tube 33' can flow to the gas mixing tube 35 through the backflow preventer 37, and the gas in the gas mixing tube 35 cannot flow into the breathing tube 33'. Because the gas mixing tube 30 may contain an inflammable gas (such as hydrogen), the backflow preventer 37 can prevent other gas in the gas mixing tube 35 from returning back to the breathing tube 33', to reduce or prevent the unfortunately ignited gas from spreading to the oxygen generator, thereby improving safety.

The location of the backflow preventer in this present disclosure can be the location described in the aforementioned embodiment, it can also be disposed in other locations. In another one of the embodiments, the backflow preventer device is disposed between the gas mixing tube and the atomized/volatile gas mixing tank, and is disposed at the entrance of the atomized/volatile gas mixing tank, to prevent the gas in the atomized/volatile gas mixing tank from backflowing to the gas mixing tube.

In this embodiment, the atomized/volatile gas mixing tank is coupled to the gas mixing tube to receive the mixed gas comprising hydrogen gas and oxygen gas. The atomized/volatile gas mixing tank 36 generates an atomized gas mixed with the mixed gas to form a healthy gas, wherein the atomized gas is one or a combination selected from a group consist of water vapor, atomized drops, and essential oils. In one embodiment, the atomized/volatile gas mixing tank 36 comprises the oscillator; the oscillator atomizes water, anatomized drops and essential oil in the atomized/volatile gas mixing tank 36 by oscillating to generate the atomized gas, and mixes the mixed gas with the atomized gas to form a health gas. According to a user's needs, the atomized/volatile gas mixing tank 36 can be selectively turned on or off to provide healthcare gas mixed with atomized gas or mixed gas (the hydrogen gas diluted by the second oxygen gas) for users to inhale.

In practice, the mixed gas generating device connected with breathing tube in the aforementioned embodiments can further comprise a control panel coupled to the above-mentioned controller, thereby controlling the electrolytic cell and adjusting the hydrogen volume concentration and the output flow rate value of the mixed gas.

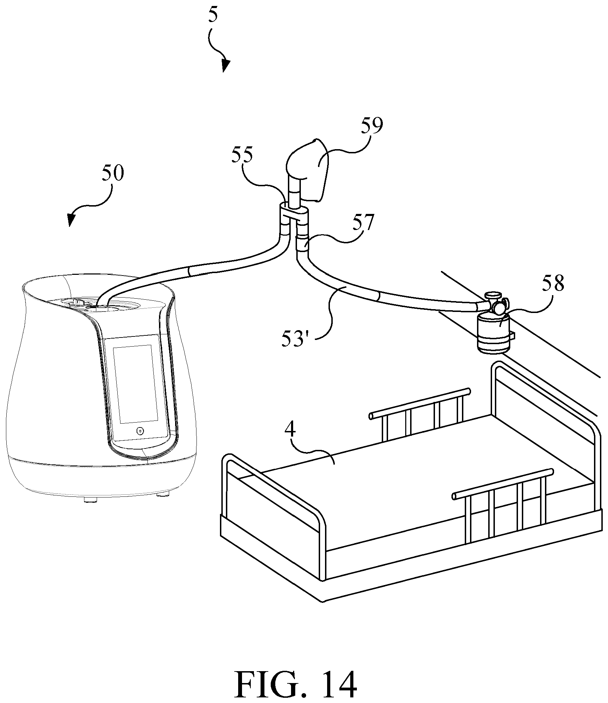

Please refer to FIG 14. FIG 14 is a schematic diagram illustrating a mixed gas generating system according to an embodiment of the present disclosure. As shown in FIG 14, the differences between this embodiment and the aforementioned embodiment are that the gas mixing tube 55, the backfire preventer 57, and the breathing tube 53' are coupled to the hydrogen generating device 50 from the outside of the hydrogen generating device 50, and the breathing tube 53' is coupled to the oxygen generating device 58 corresponding to the hospital bed 4. In this embodiment, the hydrogen generating device 50 comprises a casing, and the casing comprises an accommodating space for accommodating the electrolytic cell, the integrated sink module and the atomized/volatile gas mixing tank mentioned above. The atomized/volatile gas mixing tank comprises a gas port, and the gas mixing tube 55 is coupled to the gas port of the atomized/volatile gas mixing tank disposed outside of the casing of the hydrogen generating device 50, the breathing tube 53', and the breathing mask 59 of the hydrogen generating device 50. The breathing tube 53' is coupled to the gas mixing tube 55 and the oxygen generating device 58, and the backfire preventer 57 is disposed in the side of the gas mixing tube 55 and close to the breathing tube 53'. The electrolytic cell of the hydrogen generating device 50 generates hydrogen or gas comprising hydrogen and outputs hydrogen or gas comprising hydrogen to the gas mixing tube 55 through the atomized/volatile gas mixing tank. The oxygen generator 58 generates the first oxygen, and the first oxygen is inputted to the gas mixing tube 55 through the breathing tube 53' to form the mixed gas. Then, the user can inhale the mixed gas through the breathing mask 59.

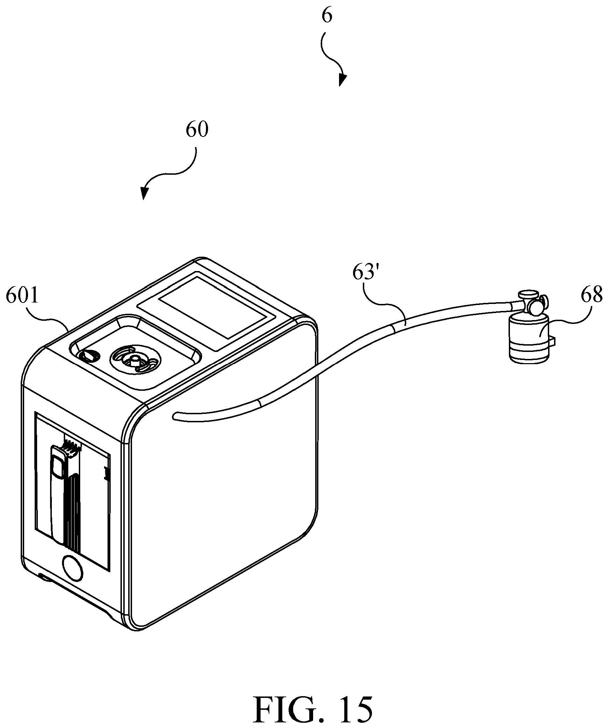

Please refer to FIG 15. FIG 15 is a schematic diagram illustrating a mixed gas generating system 6 according to another embodiment of the present disclosure. The internal structure of the mixed gas generating system 6 can refer to that in FIGs. 7 and 8. However, the difference between the internal structure of the mixed gas generating system 6 with the internal structure in FIGs. 7 and 8 is that the mixed gas generating system 6 does not have the molecular sieve filtering unit and the air pump. The other portions in the mixed gas generating system 6 are substantially the same as those in FIGs. 7 and 8, and then they would not be described again.

In this embodiment, the gas mixing tube 65 can be coupled to the exit gas channel 6042 of the integrated flow channel device 604 to receive the hydrogen gas outputted by the exit gas channel 6042. The gas mixing tube 65 is further coupled to the breathing tube 63' to receive the oxygen generated by oxygen generating device. Furthermore, the gas mixing tube 65 is configured to mix the hydrogen gas and the oxygen gas. In this embodiment, the hydrogen gas generating device 60 comprises the casing 601; the gas mixing tube 65 is disposed in the casing 601 of the hydrogen gas generating device 60; and the oxygen generating device 68 is disposed outside the hydrogen gas generating device 60. The breathing tube 63' is coupled to the oxygen generating device 68 and the gas mixing tube 65 in the casing 601 of the hydrogen generator 60 by passing through the casing 601 of the hydrogen generator 60 from the outside of hydrogen generator 60. Therefore, the oxygen generated by the oxygen generating device 68 through the breathing tube 63' into the gas mixing tube 65 to dilute the hydrogen gas to form a mixed gas. In addition, the atomized/volatile gas mixing tank 607 of the hydrogen generator 60 can receive hydrogen and oxygen in the gas mixing tube 65, and generate the atomized gas to mixed with hydrogen and oxygen to form the mixed gas. The functions of the atomized/volatile gas mixing tank of this embodiment are substantially the same as the functions of the atomized/volatile gas mixing tank of the aforementioned embodiment, which are not repeated herein. In another embodiment, the gas mixing tube and the oxygen generating device both are disposed outside of the hydrogen gas generating device. The atomized/volatile gas mixing tank is coupled to the exit gas channel of the integrated flow channel device, and the gas mixing tube is coupled to the atomizing/volatile gas mixing tank

In the summary, the mixing generating device with oxygen gas generator of the present disclosure is capable of generating oxygen gas by the oxygen gas generator and mixing the oxygen gas with the hydrogen gas produced by the electrolytic cell to dilute the hydrogen gas. The oxygen gas is mixed with the hydrogen gas to generate a mixed gas with a certain concentration of hydrogen gas for the patients with lung diseases to inhale, thus alleviating symptoms and reducing the burden on the lungs. In addition, the oxygen gas inputted by the oxygen gas generator makes the mixed gas have a higher oxygen concentration that is suitable for the patients with damaged lungs who requires high oxygen concentrations. Furthermore, the mixed gas produced by the device of the present disclosure may be mixed with the atomization gas with healing effects to form a healthy gas for human inhalation. Moreover, the device of the present disclosure can prevent the backflow of gas by backfire device, thereby improving safety. Therefore, the mixed gas generating device combined with oxygen gas generator of the present disclosure can provide gas with multiple effects at the same time, which improves the symptoms of patients with lung diseases and also can be used for general healthcare.

With the examples and explanations mentioned above, the features of the disclosure are hopefully well described. More importantly, the present disclosure is not limited to the embodiment described herein. Those skilled in the art will readily observe that numerous modifications and alterations of the device may be made while retaining the teachings of the disclosure. Accordingly, the above disclosure should be construed as limited only by the metes and bounds of the appended claims.

## Claims

1. A mixed gas generating system, comprising:
a hydrogen gas generating device (60), further comprising:
an electrolytic cell (32) configured to generate a hydrogen gas when electrolyzing water;
a condensing filter (605) configured to filter the hydrogen gas generated by the electrolytic cell; and
a humidification cup (603) configured to humidify the hydrogen gas; and
an oxygen gas generator (63) configured to generate a first oxygen gas;
wherein the hydrogen gas and the first oxygen gas are mixed to each other to form a mixed gas, wherein the mixed gas generating system further comprises a single integrated flow channel device (604), and the electrolytic cell, the condensing filter and the humidification cup are all coupled to the single integrated flow channel device, such that the hydrogen gas are transmitted from the electrolytic cell to the condensing filter and the humidification cup through the single integrated flow channel device.

2. The mixed gas generating system of claim 1, wherein the oxygen gas generator is configured outside of the hydrogen gas generating device, the oxygen gas generator comprises an oxygen gas conduit coupled to the hydrogen gas generating device to input the first oxygen gas into the hydrogen gas generating device, so as to mix the hydrogen gas and the first oxygen gas to form the mixed gas.

3. The mixed gas generating system of claim 1, wherein the oxygen gas generator comprises a molecular sieve filtering unit configured to filter an air to generate the first oxygen gas.

4. The mixed gas generating system of claim 1, further comprising a gas mixing tube coupled to the hydrogen gas generating device and the oxygen gas generator to receive the hydrogen gas and the first oxygen gas, so as to mix the hydrogen gas and the first oxygen gas to form the mixed gas.

5. The mixed gas generating system of claim 4, wherein the gas mixing tube is integrated in the integrated flow channel module of the hydrogen gas generating device or configured outside of the hydrogen gas generating device, the oxygen gas generator is configured outside of the hydrogen gas generating device.

6. The mixed gas generating system of claim 4, further comprising a backfire preventer configured between the oxygen gas generator and the gas mixing tube.

7. The mixed gas generating system of claim 1, wherein the hydrogen gas generating device further comprises a hydrogen water cup, an atomization/volatile gas mixing tank and a flame arrester, the hydrogen water cup is engaged with the integrated flow channel device and configured to accommodate water and selectively receive the hydrogen gas, the hydrogen gas flows into the hydrogen water cup to be mixed with the water contained in the hydrogen water cup to form a hydrogen water, the atomization/volatile gas mixing tank is coupled to the integrated flow channel module to receive the hydrogen gas from the integrated flow channel module, the atomization/volatile gas mixing tank selectively generates an atomized gas to be mixed with the hydrogen gas, wherein the atomized gas is one or a combination selected from a group consisting of a water vapor, anatomized drops, and an essential oils, the flame arrester is coupled to an entrance of the atomization/volatile gas mixing tank.

8. A mixed gas generating system, comprising:
a hydrogen gas generating device, further comprising:
an electrolytic cell (32) configured to generate a hydrogen gas and a oxygen gas when electrolyzing water; and
an integrated water tank module (31) comprising a hydrogen gas port (310), an oxygen gas port (312) and a water port (314) coupled to the electrolytic cell, the integrated water tank module being configured to receive the hydrogen gas and the oxygen gas from the electrolytic cell and supply water to the electrolytic cell;
an oxygen gas generator (38) physically separated from the hydrogen gas generating device and configured to generate a first oxygen gas; and
a breathing tube (33') coupled to the oxygen gas generator and configured to receive the first oxygen gas generated by the oxygen gas generator;
wherein the hydrogen gas and the first oxygen gas are mixed to each other to form a mixed gas.

9. The mixed gas generating system of claim 8, further comprising a gas mixing tube coupled to the breathing tube to mix the hydrogen gas and the first oxygen gas to form the mixed gas.

10. The mixed gas generating system of claim 9, wherein the gas mixing tube is integrated in the integrated water tank module of the hydrogen gas generating device or configured outside of the hydrogen gas generating device.

11. The mixed gas generating system of claim 8, further comprising a backfire preventer configured in the breathing tube.

12. The mixed gas generating system of claim 8, wherein the electrolytic cell further comprises a cathode chamber, a hydrogen gas output duct, an anode chamber, an oxygen gas output duct, a water input duct and an ion membrane, the cathode chamber is located on a first side of the electrolytic cell, the anode chamber is located on a second side of the electrolytic cell, and the ion membrane is disposed between the anode chamber and the cathode chamber, the oxygen gas output duct is coupled to the oxygen gas port, the hydrogen gas output duct is coupled to the hydrogen gas port, and the water input duct is coupled to the water port, the anode chamber generates the oxygen gas and the cathode chamber generates the hydrogen gas when the electrolytic cell electrolyzes water, the oxygen gas output duct is coupled with the anode chamber and penetrates the second side to output the oxygen gas at the second side, the hydrogen gas output duct is coupled with the cathode chamber and extends toward the second side and penetrates the second side to output the hydrogen gas at the second side, so as to cause the hydrogen gas and the oxygen gas to be outputted at the same side of the electrolytic cell.

13. The mixed gas generating system of claim 1, further comprising:
a breathing tube coupled to the oxygen gas generator and configured to receive the first oxygen gas generated by the oxygen gas generator which is configured outside of the hydrogen generating device.

14. The mixed gas generating system claim 13, further comprising a gas mixing tube coupled to the hydrogen gas generating device and the oxygen gas generator to receive the hydrogen gas and the first oxygen gas, so as to mix the hydrogen gas and the first oxygen gas to form the mixed gas.

15. The mixed gas generating system claim 13, wherein the gas mixing tube is integrated in the integrated flow channel module of the hydrogen gas generating device or configured outside of the hydrogen gas generating device.

16. The mixed gas generating system claim 14, further comprising a backfire preventer configured between the oxygen gas generator and the gas mixing tube.

17. The mixed gas generating system claim 13, wherein the hydrogen gas generating device further comprises a hydrogen water cup, an atomization/volatile gas mixing tank and a flame arrester, the hydrogen water cup is engaged with the integrated flow channel device and configured to accommodate water and selectively receive the hydrogen gas, the hydrogen gas flows into the hydrogen water cup to be mixed with the water contained in the hydrogen water cup to form a hydrogen water, the atomization/volatile gas mixing tank is coupled to the integrated flow channel module to receive the hydrogen gas from the integrated flow channel module, the atomization/volatile gas mixing tank selectively generates an atomized gas to be mixed with the hydrogen gas, wherein the atomized gas is one or a combination selected from a group consisting of a water vapor, anatomized drops, and an essential oils, the flame arrester is coupled to an entrance of the atomization/volatile gas mixing tank.

## Patentansprüche

1. Mischgaserzeugungssystem, umfassend:
eine Wasserstoffgaserzeugungsvorrichtung (60), die ferner Folgendes umfasst:
eine Elektrolysezelle (32), die so konfiguriert ist, dass sie bei der Elektrolyse von Wasser Wasserstoffgas erzeugt;
einen Kondensationsfilter (605), der so konfiguriert ist, dass er das von der Elektrolysezelle erzeugte Wasserstoffgas filtert; und
einen Befeuchtungsbecher (603), der so konfiguriert ist, dass er das Wasserstoffgas befeuchtet; und
einen Sauerstoffgasgenerator (63), der so konfiguriert ist, dass er ein erstes Sauerstoffgas erzeugt;
wobei das Wasserstoffgas und das erste Sauerstoffgas miteinander vermischt werden, um ein Mischgas zu bilden, wobei das Mischgaserzeugungssystem ferner eine einzige integrierte Strömungskanalvorrichtung (604) umfasst und die Elektrolysezelle, der Kondensationsfilter und der Befeuchtungsbecher alle mit der einzigen integrierten Strömungskanalvorrichtung gekoppelt sind, so dass das Wasserstoffgas von der Elektrolysezelle über die einzige integrierte Strömungskanalvorrichtung zum Kondensationsfilter und zum Befeuchtungsbecher geleitet wird.

2. Mischgaserzeugungssystem nach Anspruch 1, wobei der Sauerstoffgasgenerator außerhalb der Wasserstoffgaserzeugungsvorrichtung konfiguriert ist, der Sauerstoffgasgenerator eine Sauerstoffgasleitung umfasst, die mit der Wasserstoffgaserzeugungsvorrichtung gekoppelt ist, um das erste Sauerstoffgas in die Wasserstoffgaserzeugungsvorrichtung einzuführen, um das Wasserstoffgas und das erste Sauerstoffgas zu vermischen und so das Mischgas zu bilden.

3. Mischgaserzeugungssystem nach Anspruch 1, wobei der Sauerstoffgasgenerator eine Molekularsiebfiltereinheit umfasst, die so konfiguriert ist, dass sie Luft filtert, um das erste Sauerstoffgas zu erzeugen.

4. Mischgaserzeugungssystem nach Anspruch 1, ferner umfassend ein Gasmischrohr, das mit der Wasserstoffgaserzeugungsvorrichtung und dem Sauerstoffgasgenerator gekoppelt ist, um das Wasserstoffgas und das erste Sauerstoffgas zu empfangen, um das Wasserstoffgas und das erste Sauerstoffgas zu mischen und so das Mischgas zu bilden.

5. Mischgaserzeugungssystem nach Anspruch 4, wobei das Gasmischrohr in das integrierte Strömungskanalmodul der Wasserstoffgaserzeugungsvorrichtung integriert oder außerhalb der Wasserstoffgaserzeugungsvorrichtung konfiguriert ist, der Sauerstoffgasgenerator außerhalb der Wasserstoffgaserzeugungsvorrichtung konfiguriert ist.

6. Mischgaserzeugungssystem nach Anspruch 4, ferner umfassend eine Rückflussverhinderungsvorrichtung, die zwischen dem Sauerstoffgasgenerator und dem Gasmischrohr konfiguriert ist.

7. Mischgaserzeugungssystem nach Anspruch 1, wobei die Wasserstoffgaserzeugungsvorrichtung ferner einen Wasserstoffwasserbecher, einen Zerstäubungs-/Mischbehälter für flüchtige Gase und eine Flammensperre umfasst, der Wasserstoffwasserbecher mit der integrierten Strömungskanalvorrichtung in Eingriff ist und so konfiguriert ist, dass er Wasser aufnimmt und selektiv das Wasserstoffgas empfängt, das Wasserstoffgas in den Wasserstoffwasserbecher strömt, um mit dem im Wasserstoffwasserbecher enthaltenen Wasser vermischen zu werden, um Wasserstoffwasser zu bilden, der Zerstäubungs-/Mischbehälter für flüchtige Gase mit dem integrierten Strömungskanalmodul gekoppelt ist, um das Wasserstoffgas von dem integrierten Strömungskanalmodul zu empfangen, der Zerstäubungs-/Mischbehälter für flüchtige Gase selektiv ein zerstäubtes Gas zur Vermischung mit dem Wasserstoffgas erzeugt, wobei das zerstäubte Gas eines von oder eine Kombination, ausgewählt aus einer Gruppe bestehend aus Wasserdampf, zerstäubten Tropfen und ätherischen Ölen ist, die Flammensperre mit einem Eingang des Zerstäubungs-/Mischbehälters für flüchtige Gase gekoppelt ist.

8. Mischgaserzeugungssystem, umfassend:
eine Wasserstoffgaserzeugungsvorrichtung, die ferner Folgendes umfasst:
eine Elektrolysezelle (32), die so konfiguriert ist, dass sie bei der Elektrolyse von Wasser ein Wasserstoffgas und ein Sauerstoffgas erzeugt; und
ein integriertes Wassertankmodul (31), das einen Wasserstoffgasanschluss (310), einen Sauerstoffgasanschluss (312) und einen Wasseranschluss (314) umfasst, das mit der Elektrolysezelle gekoppelt ist, wobei das integrierte Wassertankmodul so konfiguriert ist, dass es das Wasserstoffgas und das Sauerstoffgas von der Elektrolysezelle empfängt und der Elektrolysezelle Wasser zuführt;
einen Sauerstoffgasgenerator (38), der physisch von der Wasserstoffgaserzeugungsvorrichtung getrennt ist und so konfiguriert ist, dass er ein erstes Sauerstoffgas erzeugt; und
einen Atemschlauch (33'), der mit dem Sauerstoffgasgenerator gekoppelt ist und so konfiguriert ist, dass er das erste vom Sauerstoffgasgenerator erzeugte Sauerstoffgas aufnimmt;
wobei das Wasserstoffgas und das erste Sauerstoffgas miteinander vermischt werden, um ein Mischgas zu bilden.

9. Mischgaserzeugungssystem nach Anspruch 8, ferner umfassend ein mit dem Atemschlauch gekoppeltes Gasmischrohr, um das Wasserstoffgas und das erste Sauerstoffgas zu mischen und so das Mischgas zu bilden.

10. Mischgaserzeugungssystem nach Anspruch 9, wobei das Gasmischrohr in das integrierte Wassertankmodul der Wasserstoffgaserzeugungsvorrichtung integriert oder außerhalb der Wasserstoffgaserzeugungsvorrichtung konfiguriert ist.

11. Mischgaserzeugungssystem nach Anspruch 8, ferner umfassend eine im Atemschlauch konfigurierte Rückflussverhinderungsvorrichtung.

12. Mischgaserzeugungssystem nach Anspruch 8, wobei die Elektrolysezelle ferner eine Kathodenkammer, einen Wasserstoffgasauslasskanal, eine Anodenkammer, einen Sauerstoffgasauslasskanal, einen Wasserzulaufkanal und eine Ionenmembran umfasst, die Kathodenkammer sich auf einer ersten Seite der Elektrolysezelle befindet, die Anodenkammer sich auf einer zweiten Seite der Elektrolysezelle befindet, die Ionenmembran zwischen Anoden- und Kathodenkammer angeordnet ist, der Sauerstoffgasauslasskanal mit dem Sauerstoffgasanschluss gekoppelt ist, der Wasserstoffgasauslasskanal mit dem Wasserstoffgasanschluss gekoppelt ist und der Wasserzulaufkanal mit dem Wasseranschluss gekoppelt ist, die Anodenkammer das Sauerstoffgas erzeugt und die Kathodenkammer das Wasserstoffgas erzeugt, wenn die elektrolytische Zelle Wasser elektrolysiert, der Sauerstoffgasauslasskanal mit der Anodenkammer gekoppelt ist und die zweite Seite durchdringt, um das Sauerstoffgas an der zweiten Seite auszugeben, der Wasserstoffgasauslasskanal mit der Kathodenkammer gekoppelt ist und sich zur zweiten Seite erstreckt und die zweite Seite durchdringt, um das Wasserstoffgas an der zweiten Seite auszugeben, um zu bewirken, dass das Wasserstoffgas und Sauerstoffgas auf derselben Seite der Elektrolysezelle ausgegeben werden.

13. Mischgaserzeugungssystem nach Anspruch 1, ferner umfassend:
einen Atemschlauch, der mit dem Sauerstoffgasgenerator gekoppelt ist und so konfiguriert ist, dass er das erste vom Sauerstoffgasgenerator erzeugte Sauerstoffgas aufnimmt, der außerhalb der Wasserstofferzeugungsvorrichtung konfiguriert ist.

14. Mischgaserzeugungssystem nach Anspruch 13, ferner umfassend ein Gasmischrohr, das mit der Wasserstoffgaserzeugungsvorrichtung und dem Sauerstoffgasgenerator gekoppelt ist, um das Wasserstoffgas und das erste Sauerstoffgas zu empfangen, um das Wasserstoffgas und das erste Sauerstoffgas zu mischen und so das Mischgas zu bilden.

15. Mischgaserzeugungssystem nach Anspruch 13, wobei das Gasmischrohr in das integrierte Strömungskanalmodul der Wasserstoffgaserzeugungsvorrichtung integriert oder außerhalb der Wasserstoffgaserzeugungsvorrichtung konfiguriert ist.

16. Mischgaserzeugungssystem nach Anspruch 14, ferner umfassend eine Rückflussverhinderungsvorrichtung, die zwischen dem Sauerstoffgasgenerator und dem Gasmischrohr konfiguriert ist.

17. Mischgaserzeugungssystem nach Anspruch 13, wobei die Wasserstoffgaserzeugungsvorrichtung ferner einen Wasserstoffwasserbecher, einen Zerstäubungs-/Mischbehälter für flüchtige Gase und eine Flammensperre umfasst, der Wasserstoffwasserbecher mit der integrierten Strömungskanalvorrichtung in Eingriff ist und so konfiguriert ist, dass er Wasser aufnimmt und selektiv das Wasserstoffgas empfängt, das Wasserstoffgas in den Wasserstoffwasserbecher strömt, um mit dem im Wasserstoffwasserbecher enthaltenen Wasser vermischen zu werden, um Wasserstoffwasser zu bilden, der Zerstäubungs-/Mischbehälter für flüchtige Gase mit dem integrierten Strömungskanalmodul gekoppelt ist, um das Wasserstoffgas von dem integrierten Strömungskanalmodul zu empfangen, der Zerstäubungs-/Mischbehälter für flüchtige Gase selektiv ein zerstäubtes Gas zur Vermischung mit dem Wasserstoffgas erzeugt,
wobei das zerstäubte Gas eines von oder eine Kombination ausgewählt aus einer Gruppe bestehend aus Wasserdampf, zerstäubten Tropfen und ätherischen Ölen ist, die Flammensperre an einen Eingang des Zerstäubungs-/Mischbehälters für flüchtige Gase gekoppelt ist.

## Revendications

1. Système de génération de gaz mixte, comprenant :
un dispositif générateur de gaz hydrogène (60), comprenant en outre :
une cellule électrolytique (32) configurée pour générer un gaz hydrogène lors de l'électrolyse de l'eau ;
un filtre à condensation (605) configuré pour filtrer le gaz hydrogène généré par la cellule électrolytique ; et
un récipient d'humidification (603) configuré pour humidifier le gaz hydrogène ; et
un générateur de gaz oxygène (63) configuré pour générer un premier gaz oxygène ;
dans lequel le gaz hydrogène et le premier gaz oxygène sont mélangés pour former un gaz mixte, dans lequel le système de génération de gaz mixte comprend en outre un dispositif de canal d'écoulement intégré unique (604), et la cellule électrolytique, le filtre de condensation et le récipient d'humidification sont tous couplés au dispositif de canal d'écoulement intégré unique, de sorte que le gaz hydrogène est transmis de la cellule électrolytique au filtre de condensation et au récipient d'humidification à travers le dispositif de canal d'écoulement intégré unique.

2. Système de génération de gaz mixte selon la revendication 1, dans lequel le générateur de gaz oxygène est configuré à l'extérieur du dispositif de génération de gaz hydrogène, le générateur de gaz oxygène comprend un conduit de gaz oxygène couplé au dispositif de génération de gaz hydrogène pour introduire le premier gaz oxygène dans le dispositif de génération de gaz hydrogène, de manière à mélanger le gaz hydrogène et le premier gaz oxygène pour former le gaz mixte.

3. Système de génération de gaz mixte selon la revendication 1, dans lequel le générateur de gaz oxygène comprend une unité de filtration à tamis moléculaire configurée pour filtrer un air afin de générer le premier gaz oxygène.

4. Système de génération de gaz mixte selon la revendication 1, comprenant en outre un tube de mélange de gaz couplé au dispositif de génération de gaz hydrogène et au générateur de gaz oxygène pour recevoir le gaz hydrogène et le premier gaz oxygène, afin de mélanger le gaz hydrogène et le premier gaz oxygène pour former le gaz mixte.

5. Système de génération de gaz mixte selon la revendication 4, dans lequel le tube de mélange de gaz est intégré dans le module de canal d'écoulement intégré du dispositif de génération de gaz hydrogène ou configuré à l'extérieur du dispositif de génération de gaz hydrogène, le générateur de gaz oxygène est configuré à l'extérieur du dispositif de génération de gaz hydrogène.

6. Système de génération de gaz mixte selon la revendication 4, comprenant en outre un dispositif anti-retour de flamme configuré entre le générateur de gaz oxygène et le tube de mélange de gaz.

7. Système de génération de gaz mixte selon la revendication 1, dans lequel le dispositif de génération d'hydrogène gazeux comprend en outre un récipient contenant de l'eau hydrogénée, un réservoir de mélange de gaz volatil/d'atomisation et un pare-flammes ; le récipient d'eau hydrogénée est relié au dispositif de canal d'écoulement intégré et configuré pour recevoir l'eau et recevoir sélectivement l'hydrogène gazeux, l'hydrogène gazeux pénètre dans le récipient d'eau hydrogénée pour se mélanger à l'eau qu'il contient et former ainsi une solution aqueuse hydrogénée, le réservoir de mélange de gaz volatil/d'atomisation est couplé au module de canal d'écoulement intégré pour recevoir l'hydrogène gazeux provenant du module de canal d'écoulement intégré, le réservoir de mélange de gaz volatil/d'atomisation génère sélectivement un gaz atomisé destiné à être mélangé à de l'hydrogène, dans lequel le gaz atomisé peut être un ou plusieurs gaz choisis parmi la vapeur d'eau, les gouttelettes atomisées et les huiles essentielles, le pare-flammes est raccordé à l'entrée du réservoir de mélange de gaz volatil/d'atomisation.

8. Système de génération de gaz mixte, comprenant :
un dispositif générateur de gaz hydrogène, comprenant en outre :
une cellule électrolytique (32) configurée pour générer un gaz hydrogène et un gaz oxygène lors de l'électrolyse de l'eau ; et
un module de réservoir d'eau intégré (31) comprenant un orifice de gaz hydrogène (310), un orifice de gaz oxygène (312) et un orifice d'eau (314) couplé à la cellule électrolytique, le module de réservoir d'eau intégré étant configuré pour recevoir le gaz hydrogène et le gaz oxygène de la cellule électrolytique et fournir de l'eau à la cellule électrolytique ;
un générateur de gaz oxygène (38) physiquement séparé du dispositif de génération de gaz hydrogène et configuré pour générer un premier gaz oxygène ; et
un tube respiratoire (33') couplé au générateur de gaz oxygène et configuré pour recevoir le premier gaz oxygène généré par le générateur de gaz oxygène ;
dans lequel le gaz hydrogène et le premier gaz oxygène sont mélangés pour former un gaz mixte.

9. Système de génération de gaz mixte selon la revendication 8, comprenant en outre un tube de mélange de gaz couplé au tube respiratoire pour mélanger le gaz hydrogène et le premier gaz oxygène afin de former le gaz mixte.

10. Système de génération de gaz mixte selon la revendication 9, dans lequel le tube de mélange de gaz est intégré dans le module de réservoir d'eau intégré du dispositif de génération de gaz hydrogène ou configuré à l'extérieur du dispositif de génération de gaz hydrogène.

11. Système générateur de gaz mixte selon la revendication 8, comprenant en outre un dispositif anti-retour de flamme configuré dans le tube respiratoire.

12. Système de génération de gaz mixte selon la revendication 8, dans lequel la cellule électrolytique comprend en outre une chambre cathodique, un conduit de sortie d'hydrogène, une chambre anodique, un conduit de sortie d'oxygène, un conduit d'entrée d'eau et une membrane ionique, la chambre cathodique est située d'un premier côté de la cellule électrolytique, la chambre anodique est située d'un second côté, et la membrane ionique est disposée entre les deux. Le conduit de sortie d'oxygène est raccordé à l'orifice de gaz oxygène, le conduit de sortie d'hydrogène à l'orifice de gaz hydrogène, et le conduit d'entrée d'eau à l'orifice d'eau. Lors de l'électrolyse de l'eau par la cellule électrolytique, la chambre anodique produit de l'oxygène et la chambre cathodique produit de l'hydrogène. Le conduit de sortie d'oxygène est raccordé à la chambre anodique, et pénètre dans le second côté pour émettre du gaz oxygène sur le second côté, le conduit de sortie de gaz hydrogène est raccordé à la chambre cathodique, et s'étend vers le second côté et pénètre dans le second côté pour y évacuer l'hydrogène. Ainsi, l'hydrogène et l'oxygène sont émis du même côté de la cellule électrolytique.

13. Système générateur de gaz mixte selon la revendication 1, comprenant en outre :
un tube respiratoire relié au générateur d'oxygène et configuré pour recevoir le premier gaz oxygène généré par ledit générateur de gaz oxygène, lequel est configuré à l'extérieur du dispositif de génération d'hydrogène.

14. Système de génération de gaz mixte selon la revendication 13, comprenant en outre un tube de mélange de gaz couplé au dispositif de génération de gaz hydrogène et au générateur de gaz oxygène pour recevoir le gaz hydrogène et le premier gaz oxygène, afin de mélanger le gaz hydrogène et le premier gaz oxygène pour former le gaz mixte.

15. Système de génération de gaz mixte selon la revendication 13, dans lequel le tube de mélange de gaz est intégré dans le module de canal d'écoulement intégré du dispositif de génération de gaz hydrogène ou configuré à l'extérieur du dispositif de génération de gaz hydrogène.

16. Système de génération de gaz mixte selon la revendication 14, comprenant en outre un dispositif anti-retour de flamme configuré entre le générateur de gaz oxygène et le tube de mélange de gaz.

17. Système de génération de gaz mixte selon la revendication 13, dans lequel le dispositif de génération d'hydrogène gazeux comprend en outre un récipient contenant de l'eau hydrogénée, un réservoir de mélange de gaz volatil/d'atomisation et un pare-flammes ; le récipient d'eau hydrogénée est mis en prise avec le dispositif de canal d'écoulement intégré et configuré pour recevoir l'eau et recevoir sélectivement l'hydrogène gazeux. L'hydrogène gazeux pénètre dans le récipient d'eau hydrogénée pour se mélanger à l'eau qu'il contient et former ainsi une solution aqueuse hydrogénée. Le réservoir de mélange de gaz volatil/d'atomisation est couplé au module de canal d'écoulement intégré pour recevoir l'hydrogène gazeux provenant de ce module de canal d'écoulement intégré, le réservoir de mélange de gaz volatil/d'atomisation génère sélectivement un gaz atomisé destiné à être mélangé avec l'hydrogène gazeux,
dans lequel le gaz atomisé est un gaz unique ou une combinaison de gaz choisis parmi la vapeur d'eau, des gouttelettes atomisées et des huiles essentielles, le pare-flammes est couplé à une entrée du réservoir de mélange de gaz volatil/d'atomisation.
